Europäisches Patentamt

European Patent Office

Office européen des brevets

(11) Publication number: **0 244 208 B1**

# EUROPEAN PATENT SPECIFICATION

(45) Date of publication of patent specification: 21.08.91  (51) Int. Cl.⁵: **C07C 67/48, C11B 3/08**

(21) Application number: 87303768.3

(22) Date of filing: 28.04.87

(54) Process for deodorizing triacetin produced from natural glycerin.

(30) Priority: 29.04.86 US 857224

(43) Date of publication of application:
04.11.87 Bulletin 87/45

(45) Publication of the grant of the patent:
21.08.91 Bulletin 91/34

(84) Designated Contracting States:
AT BE CH DE ES FR GB GR IT LI LU NL SE

(56) References cited:
AT-B- 189 314
DE-A- 2 900 023
DE-C- 1 082 901
US-A- 2 368 669
US-A- 3 108 133

CHEMICAL ABSTRACTS, vol. 85, no. 8, 23rd
August 1976, page 108, column 2, abstract
no. 48561r, Columbus, Ohio, US.

(73) Proprietor: CELANESE CORPORATION
1211 Avenue of the Americas
New York New York 10036(US)

(72) Inventor: Howell, Carl, J. Jr.
3944 Sussex Avenue
Charlotte North Carolina(US)
Inventor: Mc Williams, David R.
20401 Shearer Road
Davidson North Carolina(US)
Inventor: Steiner, Thomas L.
10709 Down Patrick Place
Charlotte North Carolina 28213(US)

(74) Representative: De Minvielle-Devaux, Ian
Benedict Peter et al
CARPMAELS & RANSFORD 43, Bloomsbury
Square
London WC1A 2RA(GB)

## Description

FIELD OF INVENTION

The present invention relates generally to a process whereby triacetin (i.e., glycerol triacetate) of acceptable odor quality can be produced from natural glycerin feedstock. More particularly, the present invention relates to the novel discovery that the combined action of elevated temperature and air sparging for a sufficient time period deodorizes to acceptable levels triacetin formed from the esterification of a natural glycerin feedstock.

As used herein and in the accompanying claims, the term "natural glycerin" is meant to refer to glycerin having as its origin the fats and oils of animals or vegetables. Thus, "natural glycerin" is to be understood as referring to that by-product formed as a result of the direct splitting of animal or vegetable fats and oils in the production of fatty acids of the general formula $CH_3(CH_2)_nCOOH$ or when such fats and oils are saponified in the process of manufacturing soap. "Natural glycerin" as used herein is therefore to be understood as being distinguishable from "synthetic glycerin" as the latter is intended to refer to glycerin synthesized from petroleum-based feedstocks, for example, the synthesis of glycerin from a propylene feedstock via an allyl chloride intermediate or like synthesis processes. Additionally, it should be understood that the term "glycerin" refers to purified commercial products containing greater than about 95% glycerol (i.e., 1,2,3-propanetriol) with the balance being water.

BACKGROUND OF THE INVENTION

Triacetin is widely recognized as a useful plasticizer for filamentary crimped tow, particularly cellulose acetate tow, in producing cigarette filters The procedure for producing such filters generally involves providing a tow (or untwisted bundle of several thousand filaments), crimping the tow, opening the tow to deregister adjacent crimps, fluffing the tow to permit subsequent uniform application of a plasticizer, pulling the tow through an appropriate plasticizer applicator, and thereafter treating the plasticized tow to reduce its cross-sectional size until it is approximately equal to the cross-sectional size of a tobacco column which, together with the filter, comprise a filtered cigarette. The plasticizer causes bonding of the filaments in the filter to thereby assist in forming a coherent filter rod or plug having the desired pressure drop, compressibility and like properties.

The triacetin can either itself be used as the principal active ingredient in plasticizer liquids (i.e., in concentrations of about 99% by weight or more) or can be blended with other suitable plasticizers such as, for example, triethyl citrate, dimethylethyl phthalate, or the dimethyl ether of triethylene or tetraethylene glycol, in dependence upon the type of filamentary tow being utilized and/or the resulting properties which are desired for the cigarette filter.

The production of triacetin from glycerin is very well known in this art and is practiced by the reaction of glycerin under triacetin-forming conditions with an acetic acid source (e.g. acetic anhydride). The glycerin is thus esterified to form triacetin as a reaction product. Residual acetic acid and/or acetic acid source which may be present in the reaction product stream is then subsequently removed, preferably by means of distillation. A finished triacetin product suitable for use as a plasticizer in the production of cigarette filters from crimped filamentary tow will typically be substantially free of residual acetic acid; that is, will contain less than about 0.005%, and preferably less than about 0.002% acetic acid. Additionally, the triacetin product will be substantially colorless, and will not exhibit unpleasant odors.

However, not all glycerin which is commercially available is suitable for producing triacetin of a quality demanded by filtered cigarette manufacturers. For example, glycerin which contains detectable odoriferous compounds will not be used as a feedstock for the production of triacetin since such odoriferous compounds will carry over into the resulting triacetin product which, if applied as a plasticizer during the production of cigarette filters from crimped filamentary tow, will evidence themselves in the form of an objectionable taste when the cigarette is smoked. For this reason, triacetin manufacturers have conventionally exclusively used synthetic glycerin (i.e., glycerin having its origin in and synthesized from petroleum-based compounds, for example, propylene) as their feedstock owing principally to its odor purity; that is to say its highly desirable deodorized quality.

Commercial sources of synthetic glycerin however are not plentiful, thereby creating significant problems for the triacetin producer should the commercially available supply sources of synthetic glycerin be reduced or eliminated. The available supply of natural glycerin on the other hand is relatively plentiful from numerous commercial sources and thus represents an attractive alternative to the triacetin producers' conventional use of synthetic glycerin feedstock.

Notwithstanding the alternate source (and possibly lower cost) incentive to producers of triacetin, natural glycerin has, until this invention, presented significant problems mitigating against its substitution for synthetic glycerin as a feedstock for the production of triacetin. Foremost is the problem associated with the odor quality of natural glycerin.

Because of its origin in the fats and oils of animals or vegetables, odoriferous compounds are present in natural glycerin which are not detectably present in synthetic glycerin. That is, since the fats and oils of animals and vegetables consist of both saturated and unsaturated fatty acid esters (see, "Organic Chemistry", Morrison et al, 3rd Ed., p. 1058 (1976)), residual unsaturated fatty acid esters may be present in natural glycerin owing, for example, to incomplete deesterification in the fat splitting stage and/or incomplete separation of the fatty acid esters from glycerin in subsequent refining stages employed in natural glycerin production. Likewise, unsaturated fatty acids may also be present in natural glycerin owing to incomplete separation in the refining stages during natural glycerin production. Residual unsaturated fatty acid esters and/or unsaturated fatty acids in the glycerin may then be oxidized (i.e., rancidified) at mild temperatures to form unsaturated (and possibly saturated) aldehydes which contain fewer carbons than their fatty acid precursors ("Fatty Acids (Their Chemistry, Properties, Production and Use)", Part 5, pp. 3693-3697, 3701 (1968)). Such aldehydes are highly odoriferous and may render the resulting triacetin produced from natural glycerin unusable as a plasticizer, even if present in a few parts per million. Moreover, the sources of animal or vegetable fats vary widely, thereby precluding with any degree of certainty whether the triacetin produced will have acceptable odor for use as a plasticizer.

Odor problems associated with glycerol obtained from the fats and oils of animals or vegetables and its related compounds are not new and have been the subject of various prior proposals to deodorize the same. For example, U.S. Patent No. 2,120,227 to Brant discloses deodorizing glycerol for use in regenerated cellulose film by agitating with the glycerol for at least six hours, (preferably 12-24 hours) activated charcoal and an oxidizing agent, preferably hydrogen peroxide. This oxidizing agent, in general, is disclosed as comprising any solution which yields hydrogen peroxide under the process conditions or, alternatively, ozone or oxygen under pressure. The patentee found the treatment of glycerol with hydrogen peroxide without the use of charcoal results in a product which is not improved and which, in many instances, is more odorous when used in regenerated cellulose film than glycerol which has not received such treatment. Moreover, low treatment temperatures (i.e., below $50\,^{\circ}$C) are used to avoid degradation of the hydrogen peroxide.

Lee et al in U.S. Patent No. 2,368,669 disclose purifying and deodorizing "glyceride oils"; i.e., glyceryl esters, by vaporizing unwanted volatiles (aldehydes, ketones) via distillation but with the exclusion of oxygen from the system.

Franzen et al in U.S. Patent No. 1,977,988 disclose recovering fatty acids from a crude mixture of volatile, non-aromatic hydrocarbons produced by destructive oxidation by the method of treating the mixture with a spray of water or inert organic materials at $150\,^{\circ}$ - $300\,^{\circ}$C and 1333 to 6666 Pa (10-50 mm Hg) gauge. The crude mixture is thereby freed of hydrocarboxylic acids without the formation of; e.g., unsaturated acids. The fatty acids recovered only have a slight odor due to the action of the small drops of liquid.

U.S. Patent 2,591,134 to Canariis discloses an apparatus and method for removing oxidizable taste-and-odor-producing material from liquids, such as water. Air is introduced into the liquid by means of the flow of liquid through, for example, a Venturi tube forming a part of the aeration member so that use of external sources of power; e.g., blowers, fans, compressors, etc., is avoided as well as the use of moving parts.

## BRIEF STATEMENT OF INVENTION

It is one object of this invention to provide a method to produce a triacetin product from natural glycerin feedstock which exhibits acceptable odor qualities suitable for use as a plasticizer for cellulose acetate tow to produce cigarette filters. A further object of this invention is to produce triacetin of acceptable odor quality from a feedstock of natural glycerin by an effective, commercially economic process so as to enable triacetin manufacturers to utilize the more commercially plentiful supply sources of natural glycerin as opposed to synthetic glycerin, and as such, provide a commercially economical and viable alternative to the use of synthetic glycerin as a feedstock for the production of triacetin.

These and other objects (which will become more clear to the reader after consideration of the following detailed description of the invention) are generally achieved by a process whereby natural glycerin is esterified to produce triacetin, the temperature of the triacetin is subsequently elevated while an oxygen-containing gas (i.e., air or oxygen) is sparged into and intimately mixed with the elevated temperature triacetin for a time sufficient to substantially remove odoriferous compounds therefrom. The temperature of the triacetin is elevated between about 80°C to about 130°C, and preferably between about 90° to about 100°C. The triacetin is maintained at such elevated temperature during the continuous sparging with air or oxygen for between about 10 to about 36 hours, and more preferably between about 20 to about 24 hours. The triacetin during sparging is preferably gently agitated to ensure complete and intimate contact with the air or oxygen. Additionally, the air or oxygen that is sparged into the triacetin should be substantially dry (i.e., a dew point of less than about -40°C) to avoid degradation of the triacetin, thereby ensuring that the acetic acid content after treatment by the present invention is maintained within acceptable standards (i.e., less than about 0.005%, and preferably less than about 0.002% by weight acetic acid).

Although the precise mechanism by which deodorization of triacetin is accomplished by means of the present invention is not fully understood at this time, it is surmised that the combined action of elevated temperature and air or oxygen sparging causes accelerated rancidification of residual unsaturated fatty acid esters and/or unsaturated fatty acids. The odoriferous aldehydes which are believed to be produced as a result of such accelerated rancidification are more volatile than their unsaturated fatty acid precursors since they have fewer carbon atoms per molecule. This increased volatility may therefore permit the formed aldehydes to be more readily stripped from the triacetin by means of the air or oxygen being sparged thereinto thereby resulting in a deodorized triacetin product.

## BRIEF DESCRIPTION OF THE ACCOMPANYING DRAWINGS

Reference will hereinafter be made to the accompanying drawings wherein like reference numerals throughout the various figures denote like elements, and wherein;

FIGURE 1 is a diagrammatic flowsheet showing a preferred processing sequence to produce deodorized triacetin from natural glycerin in accordance with this invention; and

FIGURE 2 is a diagrammatic flowsheet of a triacetin deodorization system which forms a part of the overall process shown in FIGURE 1.

## DETAILED DESCRIPTION OF THE PREFERRED EXEMPLARY EMBODIMENT

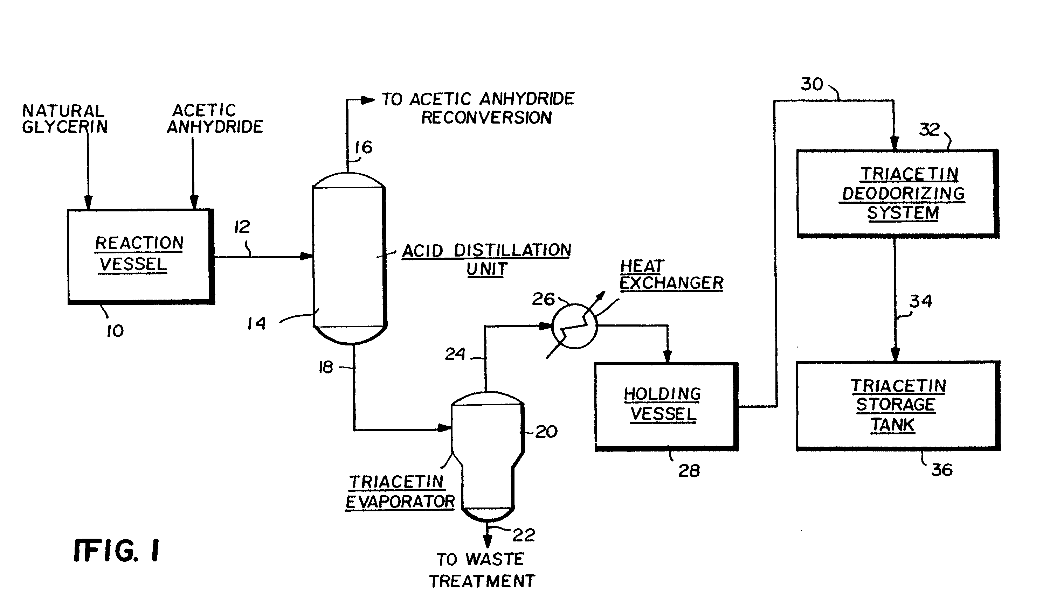

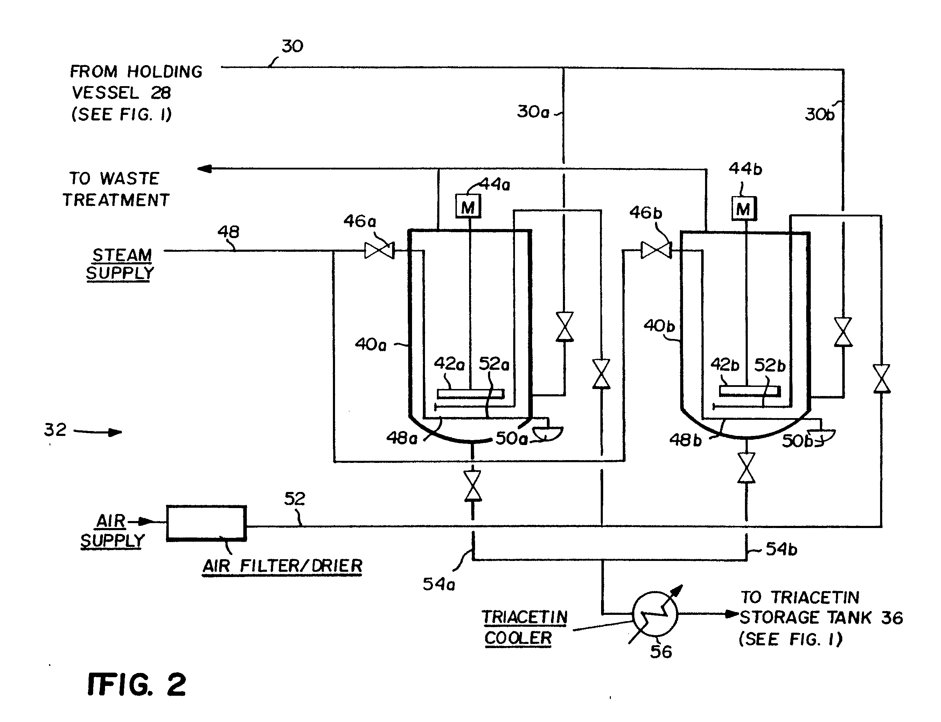

A diagrammatic flowsheet, showing a preferred processing sequence to produce deodorized triacetin from natural glycerin in accordance with this invention, is shown in accompanying FIGURE 1. As can be seen, natural glycerin and a source of acetic acid, e.g., acetic anhydride, are charged into reaction vessel 10 at a weight ratio of natural glycerin to acetic anhydride of between about .25:1 to about .29:1. The glycerin and acetic anhydride reactants are maintained in vessel 10 under triacetin-forming conditions, that is, a temperature of greater than about 130°C under atmospheric pressure for about 160 minutes. Esterification of the natural glycerin thus occurs in reaction vessel 10 so as to produce triacetin which exits in product stream 12 together with residual acetic anhydride and acetic acid formed during the reaction.

Product stream 12 then passes on to the acid distillation unit 14 whereby a substantial portion of the acetic anhydride and acetic acid is volatilized and removed in vapor stream 16 to be subsequently reconverted to acetic anhydride for use in further processing. The bottoms stream 18 from acid distillation unit 14 will thus contain substantially no acetic acid (i.e., less than about 0.005% by weight acetic acid) with the remainder being raw triacetin product. Additional purification of the raw triacetin product occurs in triacetin evaporator 20 which removes carbonaceous material and other solid particulates therefrom and passes them on to waste treatment via stream 22. The vapor stream 24 from triacetin evaporator 20 will thus be substantially free of carbonaceous material and other solid particulates and will be condensed by means of heat exchanger 26 and passed on to holding vessel 28. Raw triacetin contained in holding vessel 28 is then withdrawn as needed and passed via stream 30 to triacetin deodorizing system 32 so as to remove odoriferous compounds therefrom and thereby produce a final triacetin product acceptable for use as a plasticizer in producing cigarette filters from crimped filamentary tow. Deodorized triacetin from system 32 is then passed via stream 34 to the final product storage tank 36.

A more detailed diagrammatic flowsheet of the triacetin deodorizing system 32 can be seen by reference to accompanying FIGURE 2. The triacetin deodorizing system 32 preferably includes multiple closed treatment vessels 40a, 40b, each provided with a mechanical vane agitator 42a, 42b, driven by suitable motor means 44a, 44b, respectively. Raw triacetin is introduced into treatment vessels 40a, 40b from holding vessel 28, (see FIGURE 1) via line 30 and its manifold lines 30a, 30b. It should be understood that although two treatment vessels 40a, 40b happen to be shown in FIGURE 2, as many treatment vessels as are necessary will be required to accommodate the production of raw triacetin while yet permitting between about 10 to about 36 hours (preferably about 20 to 24 hours) resident treating time in each treatment vessel and the necessary time cycles for filling and draining of the treatment tanks. In addition, a single treatment tank could be provided without departing from the scope of this invention but is less than desirable since such a system would require a much larger holding vessel 28 and/or multiple holding vessels 28 in order to accommodate the time necessary to cyclically fill/treat/drain the triacetin utilizing a single treatment tank.

When the raw triacetin has filled either tank 40a, 40b (depending on the tank being used for a particular cycle) to a predetermined level, a corresponding steam valve 46a, 46b, respectively, is opened so as to pass steam (preferably 50 psig) from steam header 48 to steam coils 48a, 48b, located within treatment tanks 40a and 40b, respectively. In such a manner, the raw triacetin in treatment tanks 40a, 40b is heated to a temperature between about 80°C to about 130°C, and preferably to a temperature within the range of about 90° to about 100°C. Condensate is collected in traps 50a, 50b and routed to the condensate return system (not shown).

With the raw triacetin elevated in temperature and under influence of gentle agitation provided by means of mechanical agitators 42a, 42b, air or oxygen is then introduced via air supply line 52 connected to air distribution manifolds 52a, 52b within treatment tanks 40a, 40b, respectively, so as to sparge air into the heated and gently agitated raw triacetin being treated. Prior to being introduced into the treatment tanks 40a, 40b, the air or oxygen is filtered so as to remove, for example, particulate matter, oil and other contaminants therefrom and is dried to a dew point of less than about -40°C and more preferably between -40°C and -100°C. Dry air is very important to the successful practice of this invention as otherwise residual water in the air or oxygen may degrade the triacetin to form acetic acid in the treatment tanks 40a, 40b.

The triacetin is maintained at an elevated temperature as mentioned previously while air is continuously sparged thereinto via distribution manifolds 52a, 52b and is gently agitated by means of mechanical agitators 42a, 42b. Such conditions are maintained for a time period sufficient to deodorize the triacetin, and particularly for a time period between about 10 to about 36 hours (preferably between about 20 hours to about 24 hours). Sparging should be accomplished utilizing a sufficient quantity of air or oxygen to ensure that complete and intimate contact with the triacetin under treatment conditions is achieved so as to ensure adequate rancidification of residual fatty acids and/or fatty acid esters and also to ensure that the more volatile and odoriferous aldehyde compounds thus formed are entrained in the air or oxygen and removed as a vapor stream from the triacetin. Agitation of the triacetin during air or oxygen sparging will facilitate intimate contact between the air or oxygen and triacetin. Under preferred treatment conditions, at least 0.002832 m³ per hour (measured at standard temperature and pressure, that is, 25°C and 101.325 kPa) (.01 scfh) of air or oxygen per each 0.4536 Kg (per pound) of triacetin being treated (and more preferably at least 0.01416m³ per hour (.05 scfh) of air or oxygen per each 0.4536Kg (per pound) of triacetin) is sufficient to adequately deodorize the triacetin. Lesser quantities of air or oxygen may be used, however, under conditions of more vigorous agitation, for example. Moreover, excess quantities of air or oxygen may also be utilized and is limited by the practical considerations of unacceptably large quantities of triacetin product being lost due to entrainment with the air or oxygen.

The now deodorized final triacetin product is emptied from treatment tanks 40a or 40b via lines 54a or 54b, respectively, and is cooled to ambient temperatures by means of triacetin cooler 56. The cooled and deodorized triacetin is then transferred to triacetin storage tank 36 (see Figure 1) for shipment to customers for use as a plasticizer for cigarette filters. Of course, after the treatment cycle is complete and prior to emptying the triacetin from tanks 40a, 40b, the steam supply, air supply and agitation are each terminated. The deodorized triacetin may alternately be allowed to gradually cool by allowing it to stand within vessels 40a or 40b. If the triacetin is expected to stand for prolonged periods of time, it is preferred that the vessel which contains the triacetin be charged with, for example, nitrogen so as to displace substantially all air from the vessel. Such a precautionary practice ensures that the triacetin does not undergo any degenerative changes which may otherwise occur if it was maintained in contact with air for prolonged periods of time.

The present invention will be further understood from the following non-limiting examples.

EXAMPLE NO. 1

Approximately 27240Kg (60,000 pounds) of triacetin was produced in a triacetin production facility by the esterification of a natural glycerin feedstock. About 13620Kg (30,000 pounds) of the triacetin was subsequently air sparged in a pilot plant facility for 21 hours at 95°C at an air flow rate of 660 scfh. Two samples of the triacetin were obtained before the air sparging treatment (designated below as Sample Nos. 1 and 2) while one sample was obtained after the air sparging treatment (designated below as Sample No. 3). Triacetin Sample Nos. 1 through 3 produced from natural glycerin and a triacetin control sample produced from synthetic glycerin (designated below as Sample No. 4) were submitted to a panel for odor

5

evaluation. The results of the evaluation were as follows:

|                          | Sample No. | | | |
|--------------------------|------|------|------|------|
|                          | 1    | 2    | 3    | 4    |
| *Total Intensity of Aroma | ½-1  | ½    | ½    | ½    |
| Sour Buttermilk          | ½-1  | ½    | -    | -    |
| Buttermilk               | -    | -    | T    | ½    |
| Acid                     | ½    | T-½  | ½    | ½    |
| Pungent                  | ½    | T-½  | T    | T-½  |
| Overall Evaluation       | Fail | Fail | Pass | Pass |

*"T" in the tables herein designates the detection of a threshold level of odor while the numbers "½" and "1" designate increasing intensity levels, respectively, above threshold.

EXAMPLE NO. 2

Two groups of cellulose acetate cigarette filter plugs using, as plasticizer, the triacetin of Sample No. 3 and Sample No. 4, respectively, were prepared. The filter plugs of king-size filter cigarettes obtained commercially in the retail market were removed and replaced with either a filter plug using the triacetin of Example No. 3 as plasticizer or a filter plug using the triacetin of Example No. 4 as plasticizer thereby providing trial and control lots, respectively, of filtered tip cigarettes. The trial and control lots of filter tip cigarettes were then submitted to a smoking panel for evaluation in multiple, blind comparisons of the two lots of cigarettes. No consistent differences between the trial and control lots of cigarettes were detected by the smoking panel.

EXAMPLE NO. 3

Approximately 945g (750 ml) of natural glycerin was air sparged in a laboratory facility for 25.25 hours at 93-101° C at an air flow rate of 0.00708m³ per hour (0.25 scfh). Subsequent to the air sparging, 645g of the natural glycerin was reacted with acetic anhydride to form triacetin and the thus formed triacetin sample was then submitted to a panel for odor evaluation with the following results:

|                          | Result |
|--------------------------|--------|
| Total Intensity of Aroma | ½      |
| Sour Buttermilk          | ½      |
| Acid                     | T-½    |
| Pungent                  | T-½    |
| Overall                  | Fail   |

6

The above data demonstrate that triacetin produced from natural glycerin and treated according to the present invention is deodorized to a degree comparable to triacetin produced from synthetic glycerin. Particularly, it should be noted that the detected odor for the triacetin of Sample No. 3 treated according to this invention is comparable to or is less than the triacetin of control Sample No. 4, the latter representing a triacetin product now in commercial use as a plasticizer for filamentary crimped tow in the production of cigarette filters. Neither Sample No. 3 nor the control Sample No. 4 evidenced a detectable "sour buttermilk" note which is significant in determining whether triacetin is suitably deodorized for use as a tow plasticizer for cigarette filters. Without treatment according to this invention, however, a "sour buttermilk" note is detectable in the triacetin produced from natural glycerin thereby rendering such triacetin unsuitable as a tow plasticizer for cigarette filters. Moreover, Example 3 above demonstrates that treatment of natural glycerin by air sparging at elevated temperatures has no effect upon the odor quality of triacetin subsequently formed therefrom. Thus, it is only when the triacetin produced from natural glycerin is treated according to this invention that the beneficial deodorization thereof occurs to levels comparable to triacetin produced from synthetic glycerin.

While the present invention has been described in terms of what is presently considered to be the most preferred embodiment thereof, those in this art may recognize that many modifications may be made, which modifications shall be accorded the broadest scope of the appended claims so as to encompass all equivalent methods and/or processes.

## Claims

1. In a process for producing triacetin from a glycerin feedstock by means of reacting the glycerin feedstock with a source of acetic acid under triacetin-forming conditions, the improvement comprising utilizing as the feedstock natural glycerin having its origin in the fats and oils of animals or vegetables, and deodorizing the triacetin produced therefrom, wherein said step of deodorizing includes the steps of elevating the temperature of the triacetin within a range between about 80° to about 130° C and maintaining the triacetin at said elevated temperature for a treatment period between about 10 hours to about 36 hours while continuously sparging air or oxygen thereinto during the treatment period, thereby to produce a triacetin product of deodorized quality which is at least comparable to the deodorized quality of a triacetin product produced from synthetic glycerin and thus is suitable for use as a plasticizer in the production of cigarette filters.

2. A process as in Claim 1 wherein the elevated temperature of said triacetin is in the range of between about 90° C to about 100° C.

3. A process as in Claim 1 wherein said treatment period is between about 20 hours to about 24 hours.

4. A process as in Claim 1 wherein dry air is utilized in said step of sparging and has a dew point of less than about -40° C.

5. A process as in Claim 4, wherein the dry air has a dew point of between -40° C and -100° C.

6. A process as in claim 1 wherein said step of sparging is practiced utilizing air at a rate of at least 0.002832m³ per hour (0.1 scfh) for each 0.4536Kg (per pound) of triacetin.

7. A process as in claim 1 wherein said step of deodorizing further includes the step of agitating said triacetin during said treatment period to ensure complete contact between the triacetin and the air being sparged thereinto.

8. A process for deodorizing triacetin produced by the esterification of natural glycerin, said process comprising the steps of elevating the temperature of said triacetin, sparging said triacetin, while maintaining said elevated temperature thereof, with air or oxygen for a time sufficient to substantially remove odoriferous compounds therefrom whereby said triacetin is deodorized.

9. Process for producing deodorized triacetin comprising subjecting glycerin, which contains at least 95% glycerol and which is produced from the fats and oils of animals or vegetables, to esterification with a source of acetic acid to form said triacetin, elevating the temperature of the triacetin to within the range between about 80° C to about 130° C, and introducing dry air into the triacetin while maintaining said elevated temperature thereof for a period of time between about 10 hours to about 36 hours so as to remove odoriferous compounds therefrom, whereby said triacetin is deodorized to a degree at least comparable to triacetin formed from synthesized glycerin.

**10.** Process as in claim 9 further comprising agitating the triacetin concurrently with said step of introducing dry air thereinto.

**11.** Process as in claim 9 wherein said dry air has a dew point of less than -40°C.

**12.** Process as in claim 11 wherein the air is introduced into the triacetin at a rate of at least $0.002832m^3$ per hour (.01 scfh) for each 0.4536Kg (per pound) of triacetin.

**13.** Process as in claim 11 wherein the air is introduced into the triacetin at a rate of at least $0.01416m^3$ per hour (.05 scfh) for each 0.4536Kg (per pound) of triacetin.

**14.** Process as in claim 9 wherein the triacetin is elevated to a temperature within the range of about 90°C to about 100°C and is maintained at said elevated temperature for a period of time between about 20 hours to about 24 hours.

## Revendications

**1.** Procédé pour produire la triacétine à partir d'une matière première de glycérine au moyen de la réaction d'une matière première de glycérine avec une source d'acide acétique sous des conditions de formation de la triacétine, le perfectionnement qui consiste à utiliser comme matière première de la glycérine naturelle trouvant son origine dans les graisses et les huiles d'animaux ou de végétaux, et à désodoriser la triacétine produite à partir de celles-ci, la désodorisation comprenant l'étape d'élévation de la température de la triacétine dans un intervalle compris entre environ 80° et environ 130°C et le maintien de la triacétine à ladite température élevée pendant une période de traitement entre environ 10 heures et environ 36 heures tout en y faisant barboter en continu de l'air ou de l'oxygène pendant la période de traitement, ce qui permet de produire un produit de triacétine de qualité désodorisée qui est au moins comparable à la qualité désodorisée d'un produit de triacétine produit à partir de glycérine synthétique et qui est ainsi convenable pour une utilisation comme plastifiant dans la fabrication des filtres de cigarettes.

**2.** Procédé suivant la revendication 1, caractérisé en ce que la température élevée de ladite triacétine est dans l'intervalle compris entre environ 90°C et environ 100°C.

**3.** Procédé suivant la revendication 1, caractérisé en ce que ladite période de traitement est comprise entre environ 20 heures et environ 24 heures.

**4.** Procédé suivant la revendication 1, caractérisé en ce que de l'air sec est utilisé dans l'étape précitée de barbotage et présente un point de rosée de moins d'environ -40°C.

**5.** Procédé suivant la revendication 4, caractérisé en ce que l'air sec possède un point de rosée entre -40°C et -100°C.

**6.** Procédé suivant la revendication 1, caractérisé en ce que ladite étape de barbotage est mise en oeuvre en utilisant de l'air à un débit d'au moins $0,002832 m^3$ par heure (0,01 scfh) pour chaque 0, 4536 Kg (par pound) de triacétine.

**7.** Procédé suivant la revendication 1, caractérisé en ce que ladite étape de désodorisation comprend en outre une étape d'agitation de la triacétine pendant ladite période de traitement pour assurer un contact complet entre la triacétine et l'air que l'on y fait barboter.

**8.** Procédé pour désodoriser la triacétine produite par l'estérification de la glycérine naturelle, ledit procédé comprenant les étapes d'élévation de la température de ladite triacétine, de barbotage de ladite triacétine, tout en maintenant ladite température élevée de celle-ci, avec de l'air ou de l'oxygène pendant un temps suffisant pour éliminer de façon substantielle les composés odoriférants de celle-ci

grâce à quoi ladite triacétine est désodorisée.

9. Procédé pour produire de la triacétine désodorisée consistant à soumettre de la glycérine, qui contient au moins 95% de glycérol et qui est produite à partir des graisses et huiles des animaux ou végétaux, à une estérification avec une source d'acide acétique pour former ladite triacétine, à élever la température de la triacétine dans une intervalle compris entre environ 80°C et environ 130°C, et à introduire de l'air sec dans la triacétine tout en maintenant ladite température élevée de celle-ci pendant une période de temps entre environ 10 heures et environ 36 heures de façon à éliminer les composés odoriférants de celle-ci, grâce à quoi ladite triacétine est désodorisée à un degré au moins comparable à la triacétine formée à partir de glycérine synthétisée.

10. Procédé suivant la revendication 9, caractérisé en ce qu'il consiste en outre à agiter la triacétine concurremment avec l'étape précitée d'introduction d'air sec dans celle-ci.

11. Procédé suivant la revendication 9, caractérisé en ce que l'air sec précité présente un point de rosée de moins de -40°C.

12. Procédé suivant la revendication 11, caractérisé en ce que l'air est introduit dans la triacétine à un débit d'au moins 0,002832 m³ par heure (0,01 scfh) par chaque 0,4536 kg (par pound) de triacétine.

13. Procédé suivant la revendication 11, caractérisé en ce que l'air est introduit dans la triacétine à un débit d'au moins 0,01416 m³ par heure (0,05 scfh) par chaque 0,4536 Kg (par pound) de triacétine.

14. Procédé suivant la revendication 9, caractérisé en ce que la triacétine est élevée à une température dans l'intervalle compris entre environ 90°C et environ 100°C et est maintnue à ladite température élevée pendant une période de temps entre environ 20 heures et environ 24 heures.

## Patentansprüche

1. Verfahren zur Herstellung von Triacetin aus einem GlycerinBeschickungsgut durch Umsetzung des Glycerin-Beschickungsguts mit einer Essigsäurequelle unter Triacetin-bildenden Bedingungen, dadurch gekennzeichnet, daß man als Beschickungsgut natürliches Glycerin, das seinen Ursprung in tierischen und pflanzlichen Fetten und Ölen hat, verwendet und das hiervon hergestellte Triacetin desodoriert, wobei die Desodorierungsstufe die Stufen der Erhöhung der Temperatur von Triacetin in einen Bereich zwischen etwa 80° bis etwa 130°C und des Haltens des Triacetins bei dieser erhöhten Temperatur während einer Behandlungsdauer zwischen etwa 10 Stunden bis etwa 36 Stunden, während Luft oder Sauerstoff während der Behandlungsdauer kontinuierlich eingeblasen wird, umfaßt, wobei ein Triacetin von geruchloser Qualität hergestellt wird, welche mit der geruchlosen Qualität eines aus synthetischem Glycerin hergestellten Triacetinprodukts zumindest vergleichbar ist und welches sich somit für die Verwendung als Weichmacher bei der Herstellung von Zigarettenfiltern eignet.

2. Verfahren nach Anspruch 1, dadurch gekennzeichnet, daß die erhöhte Temperatur des Triacetins im Bereich zwischen etwa 90°C und etwa 100°C liegt.

3. Verfahren nach Anspruch 1, dadurch gekennzeichnet, daß die Behandlungsdauer zwischen etwa 20 Stunden bis etwa 24 Stunden liegt.

4. Verfahren nach Anspruch 1, dadurch gekennzeichnet, daß trockene Luft in der Einblasstufe verwendet wird, welche einen Taupunkt von weniger als etwa -40°C aufweist.

5. Verfahren nach Anspruch 4, dadurch gekennzeichnet, daß die trockene Luft einen Taupunkt zwischen -40°C und -100°C aufweist.

6. Verfahren nach Anspruch 1, dadurch gekennzeichnet, daß die Einblasstufe unter Verwendung von Luft bei einer Geschwindigkeit von zumindest 0,002832m³ pro Stunde (0,01 scfh) je 0,4536 kg (pro Pfund) Triacetin durchgeführt wird.

7. Verfahren nach Anspruch 1, dadurch gekennzeichnet, daß die Desodorierungsstufe weiters die Stufe des Rührens des Triacetins während der Behandlungsdauer umfaßt, um eine vollständige Berührung zwischen dem Triacetin und der eingeblasenen Luft sicherzustellen.

8. Verfahren zum Desodorieren von durch Veresterung von natürlichem Glycerin hergestelltem Triacetin, gekennzeichnet durch die Stufen der Erhöhung der Temperatur des Triacetins, des Durchblasens durch das Triacetin, unter Aufrechterhalten der erhöhten Temperatur hiervon, von Luft oder Sauerstoff, während einer Zeit, die ausreicht, um geruchsgebende Komponenten hiervon im wesentlichen zu entfernen, wobei das Triacetin desodoriert wird.

9. Verfahren zur Herstellung von desodoriertem Triacetin, dadurch gekennzeichnet, daß man Glycerin , welches zumindest 95% Glycerin enthält und aus tierischen oder pflanzlichen Fetten und Ölen hergestellt ist, einer Veresterung mit einer Essigsäurequelle unterwirft, um das Triacetin zu bilden, wobei man die Temperatur des Triacetins auf einen Bereich zwischen etwa 80°C bis etwa 130°C anhebt und trockene Luft in das Triacetin einführt, während die erhöhte Temperatur hiervon für eine Zeitdauer zwischen etwa 10 Stunden bis etwa 36 Stunden aufrechterhalten wird, um geruchsgebende Komponenten hiervon zu entfernen, wobei das Triacetin bis zu einem mit aus synthetischem Glycerin hergestellten Triacetin zumindest vergleichbaren Grad desodoriert wird.

10. Verfahren nach Anspruch 9, weiters dadurch gekennzeichnet, daß man das Triacetin gleichzeitig mit der Stufe der Einführung der trockenen Luft rührt.

11. Verfahren nach Anspruch 9, dadurch gekennzeichnet, daß die trockene Luft einen Taupunkt von weniger als -40°C aufweist.

12. Verfahren nach Anspruch 11, dadurch gekennzeichnet, daß die Luft in das Triacetin mit einer Geschwindigkeit von zumindest 0,002832m$^3$ pro Stunde (0,01 scfh) je 0,4536 kg (pro Pfund) Triacetin eingeführt wird.

13. Verfahren nach Anspruch 11, dadurch gekennzeichnet, daß die Luft in das Triacetin mit einer Geschwindigkeit von zumindest 0,01416m$^3$ pro Stunde (0,05 scfh) je 0,4536 kg (pro Pfund) Triacetin eingeführt wird.

14. Verfahren nach Anspruch 9, dadurch gekennzeichnet, daß das Triacetin auf eine Temperatur im Bereich von etwa 90°C bis 100°C gebracht und bei dieser erhöhten Temperatur für eine Zeitdauer zwischen etwa 20 Stunden bis etwa 24 Stunden gehalten wird.

NATURAL GLYCERIN    ACETIC ANHYDRIDE

→ TO ACETIC ANHYDRIDE RECONVERSION

16

REACTION VESSEL

12

ACID DISTILLATION UNIT

HEAT EXCHANGER

26

10

14

18

24

20

TRIACETIN EVAPORATOR

HOLDING VESSEL

28

22

TO WASTE TREATMENT

30

TRIACETIN DEODORIZING SYSTEM

32

34

TRIACETIN STORAGE TANK

36

FIG. 1

EP 0 244 208 B1

FIG. 2